# EUROPÄISCHE PATENTSCHRIFT

(11) **EP 1 842 536 B1**
(45) Veröffentlichungstag und Bekanntmachung des Hinweises auf die Patenterteilung: **28.11.2018**
(21) Anmeldenummer: 07104493.7
(22) Anmeldetag: 20.03.2007
(51) Int. Cl.: A61K 31/19, A61K 31/34, A61P 39/06

(54) **Verwendung von alpha-Ketoglutarsäure und 5-Hydroxy-methylfurfural zur Reduktion von oxidativem Stress**
Use of alpha-ketoglutaric acid and 5-hydroxy-methylfurfural for reducing oxidative stress
Utilisation de l'acide alpha-kétoglutarique et du 5-hydroxyméthylfurfural pour la réduction du stress oxydatif

(30) Priorität: 20.03.2006 AT 4642006
(43) Veröffentlichungstag der Anmeldung: 10.10.2007
(73) Patentinhaber: C.Y.L. Pharmazeutika GmbH, 8074 Raaba (AT)
(72) Erfinder: Moser, Peter Michael, 8200 Gleisdorf (AT); Greilberger, Joachim, 8111 Judendorf-Strassengel (AT); Maier, Alfred, 8047 Hart bei Graz (AT); Juan, Heinz, 8063 Eggersdorf (AT); Bücherl-Harrer, Christian, 8074 Raaba (AT); Kager, Ernst, 8501 Lieboch (AT)
(74) Vertreter: Vossius & Partner Patentanwälte Rechtsanwälte mbB

(56) Entgegenhaltungen:
- EP-A1- 0 326 826
- EP-A1- 1 362 586
- WO-A-2004/047832
- DE-A1- 19 929 993
- JP-A- 2000 093 096
- US-A- 5 310 768
- MATZI ET AL: "The impact of preoperative micronutrient supplementation in lung surgery. A prospective randomized trial of oral supplementation of combined alpha-ketoglutaric acid and 5-hydroxymethylfurfural", EUROPEAN JOURNAL OF CARDIO-THORACIC SURGERY, SPRINGER VERLAG, BERLIN, DE, vol. 32, no. 5, 6 October 2007 (2007-10-06), pages 776-782, XP022288111, ISSN: 1010-7940, DOI: 10.1016/J.EJCTS.2007.07.016
- SHINYA TOYOKUNI: "Oxidative Stress and Cancer: The Role of Redox Regulation", BIOTHERAPY, KLUWER ACADEMIC PUBLISHERS, DO, vol. 11, no. 2-3, 1 June 1998 (1998-06-01) , pages 147-154, XP019232366, ISSN: 1573-8280, DOI: 10.1023/A:1007934229968
- COSSEY L NICHOLAS ET AL: "Oxalate nephropathy and intravenous vitamin C.", AMERICAN JOURNAL OF KIDNEY DISEASES : THE OFFICIAL JOURNAL OF THE NATIONAL KIDNEY FOUNDATION JUN 2013, vol. 61, no. 6, June 2013 (2013-06), pages 1032-1035, ISSN: 1523-6838
- SCHEIBMEIR HEATH D ET AL: "A review of free radicals and antioxidants for critical care nurses.", INTENSIVE & CRITICAL CARE NURSING FEB 2005, vol. 21, no. 1, February 2005 (2005-02), pages 24-28, ISSN: 0964-3397

## Beschreibung

### Technisches Gebiet

Die vorliegende Erfindung ist durch die Ansprüche gekennzeichnet und beschäftigt sich mit der Reduktion und Prävention von oxidativem Stress und dessen Auswirkungen auf Mensch und Tier.

### Stand der Technik

Aus der AT 393221 Bist bekannt, dass alpha-Ketoglutarsäure und 5-Hydroxy-methylfurfural eine zerstörende Wirkung auf maligne Tumore haben, wobei das Mittel vorzugsweise als Infusion verabreicht wird.

Gerade in den letzten Jahren (ab 2000) wurde auf dem Gebiet der für den Körper (Zellen) schädlichen Substanzen auf Basis von reaktivem Sauerstoff und Stickstoff (NO, NOS, Signal-transduktion, ROS, RNS, RONS,...) viel erforscht.

Reaktive Sauerstoff-Spezies (ROS) und Reaktive Stickstoff-Spezies (RNS), kurz in RONS zusammengefasst, sind Substanzen, die durch Be-und Überlastung des Körpers durch Erkrankungen, Operationen, Verletzung, Ischämien, Reperfusion und Sport im Organismus entstehen können.

Durch neue, sensitivere Messmethodiken, welche selbst geringe Schäden in Versuchen sowohl im Körper als auch im Labor zeigen können, wurde die Erforschung dieses Bereichs erst möglich.

Die Bestimmung der Carbonylproteine ist eine in den letzten Jahren mehr und mehr aufkommende diagnostische Methode, um eine quantitative Aussage bezüglich des oxidativen Stresses aus biologischen Flüssigkeiten machen zu können. Durch Stressbelastungen jeglicher Art (Erkrankungen, Operationen, Verletzung, Ischämien, Reperfusion, Sport etc.) steigt die Belastung durch RONS mit gleichzeitiger Reduktion der antioxidativen Kapazität des menschlichen Organismus (zum Beispiel der Vitamine). Die antioxidative Kapazität ist dafür zuständig, dass keine massiven oxidativen (pathogenen) Reaktionen innerhalb des menschlichen Organismus stattfinden. Eine permanente oxidative Belastung führt zum Beispiel zu einer Modifikation von Proteinen, welche einerseits ihre Funktionen verlieren und andererseits "körperfremd" werden (wie zum Beispiel die Oxidation von LDL zu einem atherogenen LDL). Somit kommt es zu einer sehr starken Belastung des menschlichen Organismus, diese geschädigten Proteine zu katabolisieren. Weiters wird diese Belastung durch die Aktivierung des Immunsystems erhöht. Das Gleichgewicht (Oxidation/antioxidative Kapazität) verschiebt sich dadurch zugunsten der Oxidation, was kurz als "oxidativer Stress" bezeichnet wird.

Die Oxidation von Proteinen äußert sich im Anstieg von Carbonylen (Aldehyden und Ketonen), welche sich irreversibel und kovalent am Protein bilden. Eine Erhöhung des Carbonylproteingehalts bedeutet somit eine erhöhte oxidative Stressbelastung.

Jegliche Reduktion des Carbonylproteinwerts bedeutet eine Verbesserung der Stressbelastung, eine Steigerung des Allgemeinzustandes und eine Verbesserung der Lebensqualität.

Die Reduktion des oxidativen Stresses führt über sehr komplexe biochemische und pathophysiologische Vorgänge ebenso zu einer Unterstützung des Heilungsprozesses in seiner Gesamtheit, womit ihm eine synergistische Wirkung zugeschrieben werden kann.

### Darstellung der Erfindung

Die vorliegende Erfindung sieht daher alpha-Ketoglutarsäure und 5-Hydroxy-methylfurfural zur Verwendung in der Bekämpfung und/oder Prävention von oxidativem Stress bei Operationen von Menschen oder Tieren vor, wobei alpha-Ketoglutarsäure und 5-Hydroxy-methylfurfural präoperative, operativ oder postoperativ verwendet werden. Die vorliegende Erfindung betrifft außerdem alpha-Ketoglutarsäure und 5-Hydroxy-methylfurfural zur Verwendung in der Verringerung der Proteinschädigung, die durch körperliche Belastung hervorgerufen wird, bei Menschen oder Tieren. Ferner wird hierin dieVerwendung von alpha-Ketoglutarsäure und 5-Hydroxy-methylfurfural zur Herstellung eines Mittels zur Bekämpfung und/oder Prävention von oxidativem Stress bei Menschen oder Tieren beschrieben, und zwar insbesondere zur Reduktion von reaktiven Sauerstoff- und Stickstoff-Substanzen (RONS) bzw. zur Erhöhung der antioxidativen Kapazität und der damit verbundenen Wirkung auf den menschlichen und tierischen Körper.

Speziell können alpha-Ketoglutarsäure und 5-Hydroxy-methylfurfural zur Herstellung eines Mittels zur Verbesserung des Allgemeinzustandes, insbesondere für die präoperative, operative und/oder postoperative Anwendung, sowie zur Herstellung eines Mittels zur Leistungssteigerung von Menschen oder Tieren verwendet werden.

Vorzugsweise beträgt dabei das Massenverhältnis von alpha-Ketoglutarsäure zu 5-Hydroxymethylfurfural 1:1 bis 100:1.

Für die Verabreichung des Mittels sind insbesondere eine Trinklösung, ein Trinklösungskonzentrat, ein Trinkgranulat, ein Brausegranulat, eine Tablettenform oder eine intravenöse Verabreichungsform geeignete Formen.

Getränke können in flüssiger oder in fester Form (Brause, Tabletten), unverdünnt oder in durch Flüssigkeiten verdünnbarer Form (Konzentrate) vorliegen. Typische Bestandteilen pro Liter sind (wobei bei Konzentraten ein proportionales Vielfaches der Bestandteile enthalten ist):

1-10 g alpha-Ketoglutarsäure,
0,001-5 g 5-Hydroxymethylfurfural (HMF)
0,5-5 g Natriumhydrogencarbonat
1-250 g Saccharose
0,1-5 g Kaliumhydroxid
0,02-1 g Mg-Chlorid

Der pH-Wert beträgt vorzugsweise 4-6.

Das Mittel enthält eine Substanzkombination aus alpha-Ketoglutarsäure und 5-Hydroxy-Methyl-Furfural, welche entsprechend ihrer biochemischen und pathophysiologischen Wirkungen genau diese Eigenschaft zur Reduktion von reaktiven Sauerstoff- und Stickstoff-Substanzen (RONS) bei gleichzeitiger Erhöhung der antioxidativen Kapazität und der damit verbundenen Wirkung auf den menschlichen Körper auf Basis einer verbesserten Sauerstoffökonomisierung (= optimale Sauerstoffsättigung des Gewebes) zugrunde liegt. Die in Studien belegte positive Wirkung dieser Substanzkombination ist die Reduktion des oxidativen Stresses aufgrund der antioxidativen Wirkung gegenüber reaktiven sauerstoff- bzw. stickstoffhältigen Substanzen (RONS), welche insbesondere ihre pathophysiologische Wirkungsentfaltung bei Operationen (präoperativ, operativ, postoperativ), Krankheiten, Sport und Stress zeigt.

Dieselben Auswirkungen wurden bei Tieren (Pferde, Hunde, Katzen, Schweine, ...) beobachtet.

### Weg(e) zur Ausführung der Erfindung

Folgende Trinklösung wurde in klinischen Studien an Patienten prä-operativ als Trinklösung über einen Zeitraum von 12 Tagen mit einer Tagesdosis von 1 Liter fertige Trinklösung (aufgeteilt auf 500 ml vormittags und 500 ml nachmittags) ausgegeben.

8,00 g/l alpha-Ketoglutarsäure
0,93 g/l Kaliumhydroxid
4,8 g/l Natriumhydrogencarbonat
40,00 g/l Kristallzucker
0,16 g/l Magnesiumchlorid
0,8 g/l 5 HMF

Die Patienten wurden mit schweren Erkrankungen zur Operation vorgestellt. Eine konsekutive Reduktion des Allgemeinzustandes hätte eine sofortige Operation keinesfalls erlaubt.

Es zeigten sich folgende Ergebnisse:
Die Supplementierung mit dem Mittel mit einer Tagesdosis von 1 Liter zeigte in der prä-operativen Vorbereitung eine signifikante Reduktion von reaktiven Sauerstoff- und Stickstoff-Substanzen (RONS) bei gleichzeitiger Erhöhung der antioxidativen Kapazität sowie eine Verminderung des oxidativen Stresses. Als Parameter wurden die eingangs erwähnten Carbonylproteinwerte bestimmt.

Die einfache Maßnahme der Applikation des Mittels im ambulanten Bereich kann somit zu einer deutlichen Verbesserung der individuellen Ausgangssituation des Patienten vor einem geplanten chirurgischen Eingriff führen. Eine Vergrößerung der physischen Reserven des Patienten ist mit der Möglichkeit einer patientenunterstützten frühzeitigen Mobilisation und konsekutiver Reduktion der typischen postoperativen Komplikationen, welche hauptverantwortlich für lange Krankenhausaufenthalte sind, wie z.B. SIRS (Systemic Inflammatory Response Syndrome), Thrombosen, Embolien und Pneumonien, assoziiert.

Korrespondierend zum guten Allgemeinbefinden der Patienten nach der Einnahme der Trinklösung kam es zu einer massiven Reduktion der oxidativen Stressbelastung. Beachtenswert ist auch der postoperativ niedrige Spiegel an Carbonylproteinen, welcher nach einer Operation höher zu erwarten wäre. Diese Tatsache ist als Ausdruck der optimalen Scavangerfunktion des Mittels bezogen auf die entstehenden Substanzen zu werten. Diese Wirkungsweise ist besonders herauszustreichen, da bedingt durch die abgepufferten RONS Schäden an den Endothelzellen und dadurch entstehende Komplikationskaskaden verhindert werden können. Dies wird dokumentiert durch eine verkürzte Spitalsaufenthaltsdauer und weniger Komplikationen.

Dieselben Effekte konnten bei Sportlern unter extremen Belastungen wie Wettkampfbedingungen beobachtet werden, die eine Trinklösung mit folgender Zusammensetzung vor, während und nach einem Wettkampf (bis zu 2 Liter bezogen auf fertige Trinklösung pro Tag) zu sich nahmen.

6,00 g/l alpha-Ketoglutarsäure
0,4 g/l 5 HMF
4,2 g/l Natriumhydrogencarbonat
40,00 g/l Saccharose
0,6 g/l Sorbinsäure

Diese Studien zeigten die Schädigung von Proteinen an, welche durch reaktive Sauerstoff- und Stickstoff-Substanzen (RONS) verursacht wurden.

Bei Sportlern, die während der körperlichen Belastung und Regenerationsphase das Mittel einnahmen, zeigte sich, dass es zu einer Verringerung der Proteinschädigung kam. Die Sportler hatten deutlich kürzere Regenerationszeiten und konnten so die körperliche Belastung besser ausgleichen.

Bei weiteren Versuchen im Bereich des Pferdesports konnte festgestellt werden, dass eine Einnahme des Mittels vor und nach dem Einsatz bei Wettkämpfen zu einer deutlichen Verringerung der Proteinschädigung kam. Die Regenerationszeit der Tiere nach dem Wettkampf war deutlich kürzer. Bei kranken, operierten und geschwächten Tieren wurde eine verkürzte Rekonvaleszenzzeit festgestellt. Die Tiere bekamen bis zu 2 Liter des Mittels pro Tag mit folgender Zusammensetzung (1 + 5 Konzentrat):

48,00 g/l alpha-Ketoglutarsäure
0,16 g/l 5 HMF
150 g/l Natriumhydrogencarbonat
180,00 g/l Zucker
0,24 g/l Sorbinsäure
Diese Lösung wurde mit 5 Teilen Wasser verdünnt.

Versuche bei anderen Tieren (Hunde, Schweine, ...) im Bereich Belastungen bei Krankheiten und Stresssituationen (Transporte) ergaben gleiche Ergebnisse.

## Patentansprüche

1. alpha-Ketoglutarsäure und 5-Hydroxy-methylfurfural zur Verwendung in der Bekämpfung und/oder Prävention von oxidativem Stress bei Operationen von Menschen oder Tieren, wobei alpha-Ketoglutarsäure und 5-Hydroxy-methylfurfural präoperative, operativ oder postoperativ verwendet werden.

2. alpha-Ketoglutarsäure und 5-Hydroxy-methylfurfural zur Verwendung in der Verringerung der Proteinschädigung, die durch körperliche Belastung hervorgerufen wird, bei Menschen oder Tieren.

3. alpha-Ketoglutarsäure und 5-Hydroxy-methylfurfural zur Verwendung nach Anspruch 1 oder 2, **dadurch gekennzeichnet, dass** alpha-Ketoglutarsäure und 5-Hydroxy-methylfurfural in einem Masseverhältnis von 1:1 bis 100:1 vorliegen.

## Claims

1. alpha-ketoglutaric acid and 5-hydroxy-methylfurfural for use in combating and/or preventing oxidative stress in surgery of humans and animals, wherein alpha-ketoglutaric acid and 5-hydroxy-methylfurfural are used preoperatively, operatively or postoperatively.

2. alpha-ketoglutaric acid and 5-hydroxy-methylfurfural for use in reducing of protein damage in humans or animals, which is due to physical stress.

3. alpha-ketoglutaric acid and 5-hydroxy-methylfurfural for use according to claim 1 or 2, **characterized in that** alpha-ketoglutaric acid and 5-hydroxy-methylfurfural are present in a mass ratio of 1:1 to 100:1.

## Revendications

1. Acide alpha-cétoglutarique et 5-hydroxyméthylfurfural pour une utilisation dans la lutte et/ou la prévention du stress oxydatif lors d'opérations chez l'homme ou l'animal, l'acide alpha-cétoglutarique et le 5-hydroxyméthylfurfural étant utilisés en pré-opératoire, en cours d'opération et en post-opératoire.

2. Acide alpha-cétoglutarique et 5-hydroxyméthylfurfural pour une utilisation dans la réduction de l'altération des protéines provoquée par un effort physique chez l'homme ou l'animal.

3. Acide alpha-cétoglutarique et 5-hydroxyméthylfurfural pour une utilisation selon la revendication 1 ou 2, **caractérisés en ce que** l'acide alpha-cétoglutarique et le 5-hydroxyméthylfurfural se présentent en un rapport massique de 1:1 à 100:1.
